# EUROPEAN PATENT SPECIFICATION

(11) **EP 3 106 553 B1**
(45) Date of publication and mention of the grant of the patent: **25.07.2018**
(21) Application number: 15748760.4
(22) Date of filing: 27.01.2015
(51) Int. Cl.: D03D 15/00, A61F 13/00, D02G 3/22, D02G 3/32

(54) **A GARMENT WITHOUT SEAMS**
KLEIDUNGSSTÜCKE OHNE NÄHTE
VÊTEMENT SANS COUTURES

(30) Priority: 13.02.2014 ES 201430206
(43) Date of publication of application: 21.12.2016
(73) Proprietor: MLS Textiles 1992 SL., 46870 Ontinyent Valencia (ES)
(72) Inventor: LURBE MATEU, Rafael, 46870 Ontinyent (ES)
(74) Representative: Sempere Massa, Iván Luis
(86) International application number: PCT/ES2015/070052
(87) International publication number: WO 2015/121515

(56) References cited:
- EP-A1- 1 336 674
- WO-A1-00/49219
- WO-A1-2006/015599
- WO-A1-2007/134516
- WO-A1-2010/070171
- WO-A2-2007/112067
- CN-A- 102 965 792
- DE-U1-202006 006 162
- ES-A1- 2 342 589
- GB-A- 2 489 541
- EL MUNDO: 'Un calcetin que regenera and cicatriza la piel y elimina olor', [Online] 23 February 2009, XP055218892 Retrieved from the Internet: <URL:http://www.elmundo.es/elmundo/2009/02/ 23/valencia/1235394177.html> [retrieved on 2015-04-14]

## Description

### TECHNICAL FIELD

The present invention relates to a garment without seams, which can be used to be applied as a product for covering wounds, for healing said wounds and for preventing infections, eliminating odors and for other purposes.

### PRIOR ART

Hosiery garments containing chemical finishes to try to solve problems with skin breathability, scarring, bactericides or generation are known today.

The invention disclosed in patent document P200803598 claims a hosiery garment without seams primarily made from a chitin fiber-based textile structure combined with a support fiber, for example, polyamide.

In addition, it is important to mention among already disclosed spun yarns the one disclosed in utility model U200800918, relating to a type of plied spun yarn that is quite stretch resistant, has suitable flexibility and is permeable to air for absorbing moisture, keeps heat in and shows certain affinity for skin.

However, the existence of garments without seams, which are not socks, used for preventing wounds and/or healing same, is unknown.

### DISCLOSURE OF THE INVENTION

The garment without seams object of the invention makes said garments very adaptable and very durable, preventing wounds and being based on using four types of threads suitably combined with one another, said threads comprising a chitosan viscose thread, a silver-ionized polyamide thread, a third elastic thread and a fourth polyamide thread used for covering the surface of the garment produced from the spun yarn.

This invention differs from the mentioned prior art in that it provides a solution that involves the possibility of producing any type of highly adaptable garment without seams that is capable of preventing chafing and/or capable of treating wounds or abrasions.

The chitosan viscose thread incorporates ionic chitin particles that are added during its manufacturing process, such that the final spun yarn internally consists of viscose with ionic chitin particles, such that the chitin ions provide antibacterial, antimicrobial, hemostatic, antistatic, heat-regulating and therapeutically conducive properties, whereas the viscose has a high absorption capacity.

In turn, the silver-ionized polyamide thread covering an elastane element has the particularity that the silver ions give the produced garment a complete antimicrobial and antibacterial effect.

In turn, the elastic thread is preferably made from elastane and gives the garment produced by means of the spun yarn elasticity and makes it very adaptable, said elastic thread being covered by the silver-ionized polyamide thread.

Finally, the polyamide thread used as an outer covering gives the garment produced by means of the spun yarn a heat-regulating effect and makes the fabric stronger.

The arrangement of the threads is such that in the embodiment of the spun yarn, a weft of chitosan viscose thread alternates with the silver-ionized polyamide thread, these wefts joining one in two wefts of elastic thread to give elasticity to the garment that is made.

A garment without seams produced by means of the described spun yarn will thereby adapt perfectly to the body part it covers, creating optimal moisture and disinfecting conditions on wounds which help to heal small lesions on the surface of the skin.

It must also be indicated that the garment produced from the spun yarn of the invention has two-way elasticity, i.e., the garment is elastic length-wise and widthwise. This is because during the process of making it, the spun yarn made from elastane, i.e., the elastic, is positioned in circular formation around the curved body area.

This adaptability of the garment gives it wound prevention property.

The durability offered or presented by the garment produced from the spun yarn is due to the fact that said spun yarn is manufactured by addition to the raw material and not by later finishes, such as the anchor.

It should also be pointed out that the elements added during the spun yarn manufacturing process, such as silver ions or ionic chitin particles, form an inner part of the fiber by melting, which implies that the garment that is made will not undergo any type of alteration for a large number of washes. Specifically, it will withstand over 135 washes.

It must finally be pointed out that the garment made from the described spun yarn will present a considerable absorption capacity due to the presence of viscose, giving it a value of up to 440% in the laboratory testing conducted.

The following can be mentioned among the most remarkable advantages of the garment made from the spun yarn of the invention:
- It accelerates the healing process of small lesions on the skin, without leaving a scar.
- It prevents infections in superficial injuries to the epidermis of diabetic patients and in people with delicate systems due to its properties antibacterial.
- It assures the elimination of odors generated by sweat due to its antifungal properties.
- It provides a physiological feeling of comfort as it eliminates possible sources of moisture from the skin because of its considerable absorption capacity.
- It prevents dermal irritation due to contact as well as overheating.
- It prevents irritations upon contact with sensitive skin due to its biocompatibility with the skin and the technology of making it without a seam.

Based on the foregoing, the garment produced from the spun yarn of the invention is considered to be a highly recommendable garment in the health field, and specifically as a product for preventing wounds and/or for covering same for treating them.

## Claims

1. A garment without seams used for preventing wounds and/or for covering same comprising a spun yarn, **characterized in that** it comprises four types of threads: a chitosan viscose thread, another silver-ionized polyamide thread, another elastic thread, and a fourth and last polyamide thread covering the outer surface of the garment made from the produced spun yarn itself, with the particularity that said threads are arranged such that in the spun yarn, a weft of chitosan viscose thread alternates with the silver-ionized polyamide thread, said wefts joining one in two wefts of the elastic thread.

2. A garment without seams according to claim 1, **characterized in that** the chitosan viscose thread includes the addition of ionic chitin particles in its manufacturing process.

3. A garment without seams according to claim 1, **characterized in that** the elastic thread is specifically made from elastane material.

4. A garment without seams according to the preceding claims, **characterized in that** the garment made from the spun yarn resulting from the arrangement of the four threads is made with the spun yarn arranged such that two-way elasticity is obtained.

## Patentansprüche

1. Nahtfreies Kleidungsstück, welches dazu verwendet wird, Wunden zu verhindern und/oder dieselben zu decken, umfassend ein Spinnfasergarn, **dadurch gekennzeichnet, dass** es vier Arten von Fäden umfasst: einen Chitosan-Viskose-Faden, einen anderen mit Silber-ionisierten Polyamid-Faden, einen anderen elastischen Faden und einen vierten und letzten Polyamid-Faden, welcher die Außenfläche des Kleidungsstücks deckt, welches aus dem hergestellten Spinnfasergarn selbst erzeugt wird, mit der Besonderheit, dass die genannten Fäden derart angeordnet sind, dass sich im Spinnfasergarn ein Schuss von Chitosan-Viskose-Faden mit dem mit Silber-ionisierten Polyamid-Faden abwechselt, wobei die genannten Schüsse sich mit jedem zweiten Schuss des elastischen Fadens verbinden.

2. Nahtfreies Kleidungsstück nach Anspruch 1, **dadurch gekennzeichnet, dass** der Chitosan-Viskose-Faden das Hinzufügen von ionischen Chitin-Partikeln in dessen Herstellungsprozess beinhaltet.

3. Nahtfreies Kleidungsstück nach Anspruch 1, **dadurch gekennzeichnet, dass** der elastische Faden spezifisch aus Elasthan-Material erzeugt ist.

4. Nahtfreies Kleidungsstück nach den vorhergehenden Ansprüchen, **dadurch gekennzeichnet, dass** das Kleidungsstück erzeugt aus dem Spinnfasergarn, welches aus der Anordnung der vier Fäden resultiert, mit dem Spinnfasergarn derart angeordnet erzeugt wird, dass eine bidirektionale Elastizität erhalten wird.

## Revendications

1. Vêtement sans coutures utilisé pour éviter des blessures et/ou les recouvrir comprenant un fil textile filé, **caractérisé en ce qu'**il comprend quatre types de fils: un fil de chitosan-viscose, un autre fil de polyamide ionisé d'argent, un autre fil élastique, et un quatrième et dernier fil de polyamide recouvrant la surface extérieure du vêtement réalisé à partir du propre fil textile filé produit, avec la particularité que lesdits fils sont aménagés de manière que dans le fil textile filé, une trame de fil de chitosan-viscose alterne avec le fil de polyamide ionisé d'argent, lesdites trames unissant une de chaque deux trames du fil élastique.

2. Vêtement sans coutures selon la revendication 1, **caractérisé en ce que** le fil de chitosan-viscose comprend l'addition de particules de chitine ioniques dans son procédé de fabrication.

3. Vêtement sans coutures selon la revendication 1, **caractérisé en ce que** le fil élastique est réalisé spécifiquement à partir de matière d'élasthanne.

4. Vêtement sans coutures selon les revendications précédentes, **caractérisé en ce que** le vêtement réalisé à partir du fil textile filé résultant de l'aménagement des quatre fils est réalisé avec le fil textile filé aménagé de manière que l'on obtient une élasticité bidirectionnelle.
